(19) Europäisches Patentamt
European Patent Office
Office européen des brevets

(11) Publication number: **0 055 702**
**B1**

(12) # EUROPEAN PATENT SPECIFICATION

(45) Date of publication of patent specification: **08.04.87**

(51) Int. Cl.$^4$: **A 61 K 33/04**

(21) Application number: **82200091.5**

(22) Date of filing: **29.06.81**

(60) Publication number of the earlier application in accordance with Art. 76 EPC: **0 043 274**

(54) **Antithrombotic treatment.**

(30) Priority: **30.06.80 US 164845**
**22.01.81 US 227382**
**16.06.81 US 271850**

(43) Date of publication of application:
**07.07.82 Bulletin 82/27**

(45) Publication of the grant of the patent:
**08.04.87 Bulletin 87/15**

(84) Designated Contracting States:
**AT BE CH DE FR GB IT LI LU NL SE**

(56) References cited:
**FR-M-2 613**
**GB-A-816 592**
**US-A-3 279 993**

**DICTIONNAIRE VIDAL, 1968, O.V.P. PARIS (FR)**
**DICTIONNAIRE VIDAL, 1979, O.V.P. PARIS (FR)**
**DICTIONNAIRE VIDAL, 1971 O.V.P. PARIS (FR)**
**ROTE LISTE, 1st August 1979 editor Cantor AULENDORF/WÜRTTEMBERG (DE)**
**UNLISTED DRUGS, volume 27, no. 9, September 1975, CHATHAM, NEW YORK (US)**

(73) Proprietor: **T AND R CHEMICALS, INC.**
**700 Celum Road**
**Clint Texas 79836 (US)**

(72) Inventor: **Arias Alvarez, Jose Antonio**
**Carpatos 705**
**Mexico 10 (MX)**

(74) Representative: **Burford, Anthony Frederick**
**W.H. Beck, Greener & Co. 7 Stone Buildings**
**Lincoln's Inn**
**London WC2A 3SZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 055 702 B1**

**Description**

Sodium bisulphite (usually shown by formula to be $NaHSO_3$) has heretofore been used for many commercial purposes, such as a preservative for prevention of the deterioration of liquids such as food stuffs and pharmaceutical solids, and has been used medically externally for parasitic skin diseases and internally as a gastrointestinal antiseptic and for solubilizing kidney stones. It is not known to be of use in antithrombotic therapy.

The solid sodium bisulphite of commerce reportedly consists chiefly of sodium metabisulphite, $Na_2S_2O_5$, and sodium bisulphite, and, for purposes of this invention, such is believed to possess the same properties as (and to be equivalent to) sodium bisulphite when dissolved in an aqueous solution. The term "bisulphite" as used herein includes metabisulphites except where it is clear that true bisulphite is intended.

Sodium sulphite is known as an antiseptic and potassium sulphite is known as a cathartic and diuretic. They are not known to be of use in antithrombotic activity.

Reiss and Gerstl (Exptal. Med. Surg. Vol. 4, 1946, 33—347) disclose the injection into rabbits of 0.6M (about 6% by wt) sodium bisulphite aqueous solution when comparing the systemic effects of thioglycolic acid and sodium bisulphite. There is no reference to any antithrombotic activity of either compound.

Kikugawa and Iizuka (J. Pharm. Sci., Vol. 61, No. 12, December 1972 at pages 1904 to 1907), Chao, Tullis, Conneely and Lewler (Thromb Haemostasis, Vol. 35, No. 3 at pages 717—736) and Elias and Iyer (Thromb Diath Haemorrh, Vol. 18 at pages 499—509) relate to the effect of sodium bisulphite and sulphite upon blood. However, it is essential to note that none of them are concerned with assessing any therapeutic use of sodium bisulphite/sulphite and that they are all concerned with *in vitro* tests relating to the study of blood mechanisms. As explained below, they all report activity only at dose levels which when scaled up to adult human size would be totally unacceptable for therapeutic purposes.

Kikugawa describes the results of an investigation into the effect of sodium bisulphite/sulphite on blood platelet aggregation. The work was carried out because of the use of bisulphite as an antioxidant in injections and as an antiseptic. The concern of Kikugawa and others at that time was the toxic and not therapeutic activity of sodium bisulphite. In particular, there is no reference to therapeutic use and the conclusion in the last paragraph of the paper is that sodium bisulphite "might be an excellent tool for the investigation of the mechanisms of platelet aggregation and its inhibition".

In the tests reported on page 1905 under the heading "Platelet Aggregation" the effect of sodium bisulphite on platelet aggregation induced by adenosine diphosphate (ADP) was investigated. The results are given in Table I and Figure 1. It can be seen that there was little inhibition at 1 mM and that even at 5 mM there was only 66% inhibition. A concentration of 1 mM is about 100 mg/L and, in the average 70 kg adult human having 5 litres of blood, would represent an amount of about 500 mg in the blood. The blood constitutes about 10% of the total body fluid in a human and it is expected that sodium bisulphite/sulphite would rapidly be disseminated throughout the body even when administered by injection. Thus, the actual dose required to be administered to the adult human to provide a 1 mM concentration of sodium bisulphite in the blood would be substantially greater than 500 mg and probably about 5 g. However, at the 1 mM level only 9% inhibition was observed. In order to produce a 66% inhibition, a concentration of 5 mM was required and, for the reasons explained above, this corresponds to a dose in the adult human of substantially more than 2.5 g and probably about 25 g. At the 10 mM level, producing 69% inhibition, the corresponding adult human dose is at least 5g and probably about 50 g.

In connection with the above, it is not simply necessary to scale up the animal dose to a 70 kg body weight level in order to determine the equivalent human dose. However, as far as we know, there was no information available prior to the present invention that would enable any meaningful comparison to be made between the animal and human doses. Accordingly, all that can be done is to provide a scaled up amount.

Further tests reported on page 1905 appear under the headings "Deaggregation", "Effect of Treatment of Adenosine Diphosphate with Bisulphite-Sulphite on Platelet Aggregation", "Effect of Treatment of Platelets-Poor Plasma with Bisulphite-Sulphite on Platelet Aggregation", and "Effect of Bisulphite-Sulphite on Platelet Aggregation in Presence of Other Blood Cells". The results of these tests are set forth in Tables II, III, IV and Figures 2 and 3. Each of these tests was conducted at a concentration of 7.5 mM. As indicated above, this corresponds to a dose level in the adult human of at least 3.75 g and probably 37.5 g.

The experiments were carried out using rabbit blood. The LD50 (i.e. dose level at which 50% of rabbits die) is 65 mg/kg. 1 mM corresponds to about 100 mg/kg, assuming that all the sodium bisulphite/sulphite is present in the blood. Accordingly, it would appear that the lowest concentration used in the work conducted by Kikugawa was at a level above the LD50 of rabbits.

Having regard to the above, the experimental results reported by Kikugawa certainly do not teach the skilled man that the inhibition of platelet aggregation caused by sodium bisulphite/sulphite would have any therapeutic use. The inhibition appears to exist only at levels above the toxic dose in the animal model being used. Further, the doses which would appear to be required for the treatment of the adult human (assuming that anyone would consider this possibility upon reading Kikugawa) are so high as to be unacceptable. Moreover, there is certainly no teaching in Kikugawa which would lead the reader to expect that an antithrombotic activity would be exhibited at the dose levels to be used in accordance with the

present invention (see below). The dose levels are 1 to 100 mg/kg with 20 go 50 mg/kg being preferred. In an average adult human of 70 kg weight, this corresponds to a dose of 70 mg to 7 g per day.

In connection with the PT and PTT results reported below, it is significant to note that Kikugawa states at the end of the penultimate paragraph of the paper that at 10 mM sodium bisulphite/sulphite concentration, no effect on blood coagulation time was observed.

Chao is concerned with investigating the involvement of thrombin in the aggregation of platelets and inert particles, particularly, in respect of the relationship between platelet-polymerizing fibrin interaction and platelet aggregation. Part of the investigation involved study of the inhibition of aggregation by sodium sulphite. In this work human blood was used. A conclusion, which appears to be independent of the inhibitory effect of sodium sulphite, is that blood coagulation mechanism may be directly involved in platelet aggregation.

It is reported on page 723 under the heading "Effect of Sodium Sulphite" that 10 to 50 mM sodium sulphite inhibited thrombin-induced light transmission increase (LTI) in a celite-fibrinogen suspension but had no effect on the first wave of platelet-fibrinogen preparations. A concentration of 10 to 50 mM sodium sulphite corresponds to an amount of about 6 to about 30 g in the blood of an adult human which probably would require administration of a dose of about 60 to about 300 g. As indicated in Figure 5, there is only partial inhibition at the 10 mM level with complete inhibition occurring at the 50 mM level.

The "no effect level" specified by the US Federal Drug Agency in relation to the use of sodium bisulphite/sulphite in foods is only 30 to 100 mg/kg sulphur dioxide. This corresponds to an amount of about 45 to about 150 mg/kg sodium bisulphite. In the adult human (70 kg) this corresponds to about 3 g to about 10 g, of which about 300 mg to about 1 g would be in the blood.

There is no suggestion in Chao that the anti-aggregation properties of sodium sulphite could be of therapeutic use. Not only are the concentration of sodium sulphite used well above those which would be considered for therapeutic purposes but also the experiment was concerned with a celite-containing suspension and not blood alone.

Elias is concerned with too inhibition of fibrinogen-fibrin conversion. Thus, it is concerned with the last stage in the blood coagulation cascade. Fibrinogen is blood clotting Factor 1.

The "thrombin time" is the time interval between the addition of thrombin to a fibrinogen solution and the gel point. Sodium sulphite was one of several substances investigated and was found to be less effective than thiols and EDTA. The tests were carried out using dog plasma and the effect on thrombin time is set forth in Table I on page 503. At a concentration of 10 mM, sodium sulphite increased the thrombin time by about 38%. As mentioned previously, a concentration of 10 mM in the adult human is about 6 g in the blood and corresponds to a probable dose of 60 g.

Other effects of sodium sulphite were tested and are reported on pages 503 to 505 under the headings "Effect of Inhibitors on TAME Esterolytic Activity of Thrombin", "Effect of Preincubation of Fibrinogen with Inhibitors on the Enzymatic Phase", "Polymerizing of Fibrin Monomer" and "Opacity Changes during Polymerization". No inhibitory effect on TAME esterolytic activity of thrombin was observed. The results for the other effects are given in Table II, Table III and Figure 6 respectively. The results in Table II and Figure 6 at a concentration of 10 mM sodium sulphite, the same concentration as that at which inhibition of thrombin time was measured. However, the polymerization of fibrin monomer results given in Table III are at 100 mM sodium sulphite concentration corresponding to a blood content in the adult human of about 60 g and a probable dose of 600 g.

Once again, the concentration levels used in the experimental work are so high that they have no relevance to the possible therapeutic use of sodium sulphite. Thus, not only is there no suggestion that there could be therapeutic use but the reported concentrations teach against the possibility of such use.

In connection with the Elias paper, it is important to note that the PT and PTT measurements relate to much earlier stages in the blood coagulation cascade than the fibrinogen-fibrin conversion. In general terms, it is more advantageous to influence blood coagulation at an early stage in the cascade than it is at the last stage. If blood coagulation is inhibited at the last stage, it is difficult to correct an overdose situation or to stop bleeding if it occurs. On the other hand, if the cascade is inhibited at an earlier stage, the option of inducing coagulation at a later stage is available and therefore, in general terms, the inhibitor is safer in use.

Anticoagulants and antithrombotic agents are a group of compounds with diversified pharmacological actions which are used in a variety of clinical thrombotic disorders. Thrombotic disorders are generally divided into venous thrombosis and arterial occlusive disorders. Venous thrombosis of the lower extremities is important, because it can ccause pulmonary embolisms which may be fatal. "Heparin" and "Warfarin" are commonly used in clinical medicine for the prevention and treatment of deep venous thrombosis and pulmonary embolisms. Their main pharmacological actions are to inhibit or interrupt blood coagulation activity.

Platelets play an important part in arterial thrombosis. Drugs which inhibit platelet aggregation are generally regarded as potentially useful for the prophylatic therapy of arterial thrombotic disorders, including, for example, strokes, myocardial infarctions and peripheral vascular disease. Despite the availability of many agents which possess platelet anti-aggregatory properties, only a few are current under clinical trial (for example acetylsalicylic acid, dipyridamole and sulphinpyrazone). None of these

agents exhibits unequivocal efficacy. Compounds with more specific pharmacological actions are urgently sought in order to provide better medical care for patients with these serious disorders.

An anticoagulant is a substance which inhibits coagulation of the blood. A platelet anti-aggregatory agent is a substance which inhibits platelet aggregation.

An antithrombotic agent is a substance which inhibits the formation or development of a thrombus (or thrombosis). For present purposes, the term "thrombus" or its equivalent includes the term "embolus", unless otherwise specifically indicated. In general, an antithrombotic agent, in the presence of mammalian blood or appropriately-prepared plasma, may display anticoagulant activity and/or platelet anti-aggregatory activity.

Examples of clinical thrombotic conditions include stroke (as a cerebral vascular thrombosis), myocardial infarction (coronary artery disease), peripheral vascular disease, cardiac valve replacement, deep vein thrombosis and pulmonary embolism.

A class of active agents has now been discovered the members of which, when orally ingested and/or injected, produce amelioration of a thrombotic condition in mammals (including man), when used in anti-thrombotically-effective amounts as described below.

The mechanism(s) by which the active agents function is/are currently unknown; however, an inhibition of platelet aggregation and a prolongation of normal blood coagulation time appear to be associated with their use, in the manner disclosed herein.

Our copending European Patent Application No. 81302950.1 (Publication No. 0043274), from which the present disclosure has been divided, relates to the preparation and use of groups of compounds (and compositions containing them), which are within the class of active compounds in question. One aspect of the invention described and claimed in Application No. 81302950.1 (EP—A—43274) is a pharmaceutical composition which contains at least one carrier substance and, as the active treating agent, at least one compound selected from:

(A) compounds of the formula:

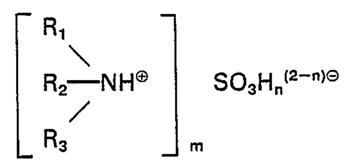

wherein:

$R_1$ is an alkyl radical containing less than 11 carbon atoms, a cycloalkyl radical containing 6 to 10 carbon atoms or a monohydroxyalkyl radical containing less than 11 carbon atoms,

$R_2$ is a hydrogen atom, an alkyl radical containing less than 11 carbon atoms or a monohydroxyalkyl radical containing less than 11 carbon atoms, or

$R_1$ and $R_2$ together form an unsubstituted morpholine, piperidine or hexamethyleneimine ring or such a ring carrying on one of its carbon atoms an alkyl radical containing less than 11 carbon atoms,

$R_3$ is a hydrogen atom, an alkyl radical containing less than 11 carbon atoms, a monohydroxyalkyl radical containing less than 11 carbon atoms or a radical of the formula:

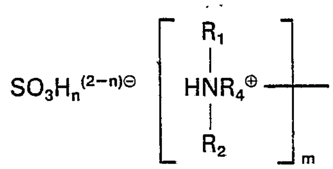

wherein $R_1$ and $R_2$ have the meanings defined above,

$R_4$ is a divalent saturated aliphatic radical containing less than 11 carbon atoms, and

m is 1 or 2, n is 0 or 1 and the sum of m and n is 2;

compounds of either of the formulae:

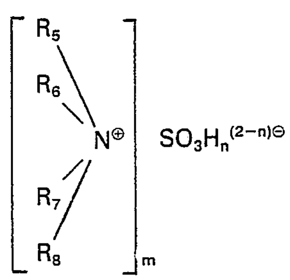

and

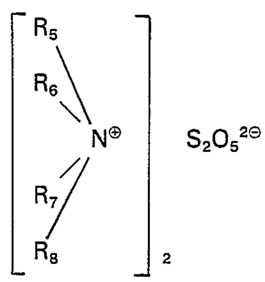

wherein:

$R_5$, $R_6$, $R_7$ and $R_8$ are the same or different and each is an alkyl radical containing less than 11 carbon atoms, and

m and n have the meanings defined above;

(C) compounds of the formula:

$$R—CHOH—SO_3M$$

wherein:

R is a hydrogen atom or a hydroxymethyl radical and

M is an alkali metal or an ammonium group, but excluding:

(a) 0.6M injectable aqueous solutions of ethanolamine bisulphite, and

(b) compositions containing a formaldehyde bisulphite and a protamine.

The present invention concerns other groups of active treating agents within the afore-mentioned class of active agents.

In accordance with this invention, a pharmaceutical composition for treating an actual or incipient thrombotic condition in a mammal is characterized in that it contains at least one carrier substance and, as the only active treating agent, at least one inorganic compound selected from alkali metal, alkaline earth metal and ammonium sulphites and bisulphites and sulphurous acid and the composition is in the form of:—

(a) a gelatin capsule, or

(b) an aqueous solution containing 1 to 10% of the active treating agent contained in a drop dispenser.

Preferably, the active treating agent is sodium bisulphite.

In one aspect of the present invention is concerned with certain bisulphite and sulphite pharmaceutical compositions for use as antithrombotic, anticoagulant and platelet anti-aggregatory agents in mammalian medicine (including human medicine). These agents are believed to be usable in both arterial and venous thrombotic conditions.

In another aspect, the compositions of the present invention can be used for the control of and/or the prevention of an embolus or a thrombus in man by oral ingestion and/or by injection of a pharmaceutically-effective amount of one or more compounds comprising the active agents of this invention.

In another aspect, the compositions of the present invention can be used for the symptomatic and objective improvement in thrombotic (including cardiovascular) disease conditions, for example, abnormal coagulation or intravascular thrombosis, in man. The term "symptomatic improvement", as used herein, means an improvement in a patient's subjective symptoms (e.g., as reported by the patient). The term "objective improvement", as used herein, means a measurable change in a patient's condition.

More particularly, the compositions of this invention can be used for treating a human or other mammal wherein there is introduced orally and/or by injection into such mammal a pharmaceutically-effective amount of an active agent of this invention as an antithrombotic agent.

Sulphite and/or bisulphite anions do not normally occur in human tissues or blood, so far as is now known.

In medicine, arterial thrombosis is diagnosable, for example, by clinical manifestations, by arteriography and, recently, by an indium 111 platelet labelling technique (see, for example, the article entitled "Differential Effects of Two Doses of Aspirin on Platelet-Vessel Wall Interaction In Vivo" by K. K. Wu et al being published in the Journal of Clinical Investigation, August, 1981).

Also, a thrombosis is detectable, for example, from a patient's conditions symptomatically perceivable by a skilled medical practitioner and well known to the art of medicine. Objectively, various methods are available, including venography, impedance plethysmography, doppler ultrasound and the $^{125}$I-fibrinogen test (see, for example, the articles by Kakkar, "Archives of Surgery", 104, page 152 (1972) and by Kelton, J.G. et al, Journal of Clinical Investigation, Vol. 62, pgs. 892—895, (1978)).

The present invention does not contemplate feeding a normal patient (that is, one not suffering from a thrombotic condition) an active agent of this invention at a pharmaceutically-effective dosage as indicated herein.

The term "thrombotic condition" as used herein, means both:

(a) an existing thrombus (including an embolus);

(b) an incipient thrombus (including an incipient embolus).

An incipient "thrombus" or "incipient thrombotic condition", as used herein, is a condition which can exist in a patient who is predisposed to the development of a thrombotic condition. For example, diabetes mellitus and hyperlipidemia are conditions which predispose a patient to arterial thrombosis. On the other hand, surgery, trauma and bed rest, for example, predispose a patient to venous thrombosis.

Those skilled in the practice of medicine routinely determine the presence of a thrombotic condition (including an actual thrombus in a patient).

Preferably, the administration of the compositions of this invention in vivo involves introducing into the blood of a patient such as a human, the equivalent of 1 to 100 milligrams per kilogram of mammal body weight (including human) per day, though larger or smaller dosage rates may be employed, if desired. The exact amount or dose in any given case is selected to be sufficient and appropriate for achieving a desired antithrombotic effect.

In general, such an introduction may be commenced at a dosage rate within the range above indicated as soon as a thrombotic condition (or a thrombus) is found in a patient.

For example, it is preferred that as a first step, a determination is made that a patient suffers from a thrombotic condition. Then, one starts orally feeding and/or injecting such patient with at least one active agent of the present invention at an effective dosage rate in the range indicated above. Currently, an especially preferred dosage rate is 20 to 50 mg/kg per day. Preferably, at least two or three spaced doses per day are given, each such dose being conveniently administered around a meal-time. Any convenient dosage arrangement can be employed.

Not uncommonly, it is desirable or necessary to start treatment immediately upon the discovery of a patient's thrombotic condition, in order to avoid damage or injury or perhaps even death of the patient, as from an embolus. If oral administration is not convenient or rapid enough, the active agent can be directly introduced by injection into the patient, if desired, such as intravenously, intramuscularly or subcutaneously. When an active agent is directly introduced, it is preferably dissolved in an aqueous medium, the total amount of active agent introduced into such medium preferably being within the range from 1 to 11 weight precent (based on the total solution weight). Distilled water is preferred as the aqueous medium. If desired, conventional (standardized) aqueous media can be used as vehicles for such introduction; for example, standard saline solutions can be used as vehicles.

In order to evaluate an antithrombotic effect, it is preferable to withdraw samples of blood from a patient undergoing treatment and measure platelet aggregation. One method is described by Born in Nature 194, pp. 927—929 (1962) and may be used for this purpose, if desired.

After administration has started, the dosage rate is preferably adjusted to a value which is sufficient to disrupt platelet function and/or coagulation factors and thereby achieve a desired antithrombotic effect.

An active agent of this invention is characteristically capable of exhibiting platelet aggregation both in vitro and in vivo. Also, such an active agent is characteristically capable of lengthening both PT (prothrombin time) and PTT (blood partial thromboplastin time) in vitro. The dosage rate of the active agent is currently believed to be related to the resulting effects upon blood factors, such as inhibition of platelet aggregation. Consequently, under this preferred procedure, use of an active agent at a suitable dose for an individual patient ameliorates that patient's thrombotic condition.

Selected blood parameters of a patient are preferably determined before dosing with an active agent is started, when time permits. Preferably, dosage rate adjustment is made while administration of an active agent is continuing. The amount of adjustment (or incremental change in dosage) is determinable by comparing a patient's measured values during administration of active agent to desired values (such as the patient's own corresponding starting values or normal species, e.g. human, values). Inhibition of platelet aggregation can be used for such measurements. Then, the deviation, if any, from the patient's measured values is compared to the desired values (the patient's starting values or normal species values, for example). Then, a change in dosage rate may be made to correct any deviation so determined.

For instance, in humans, normal values for platelet aggregation are dependent upon the particular agent used for stimulation. For example, when adenosine diphosphate (ADP) at 3 micromolar concentration is employed, platelet aggregation values fall typically in the range from 50% to 100% of light transmission. Other stimulation agents include collagen, epinephrine and arachidonic acid.

Also, in humans, normal PT values fall in the range from 11 to 13 seconds, while normal PTT values fall in the range from 25 to 41 seconds. If PT values and/or PTT values could be measured in a given patient, for the purpose of achieving a desired antithrombotic effectiveness, it is currently estimated that a lengthening of PTT value to 1.5 to 2 times a PTT value in such normal range in a given starting patient is appropriate or suitable for antithrombotic effectiveness, which amounts to a PTT value for a given patient of 45 to 60 seconds; such an estimate is consistent, for example, with the lengthened PTT values achieved in the human use of heparin, sometimes employed previously as an antithrombotic agent. Similarly, it is currently estimated that a lengthening of PT value to 2.0 times a PT value in such normal range in a given starting patient is appropriate or suitable for antithrombotic effectiveness, which amounts to a PT value for a given patient of 22 to 26 seconds; such an estimate is consistent, for example, with the lengthened PT values achieved in the human use of coumadin (Warfarin), sometimes employed previously as an antithrombotic agent. The active agents of the present invention, contrary to such prior art agents, surprisingly appear to effect both PT and PTT values. The mechanism by which the present active agents work is apparently substantially different from, and not comparable to, the prior art agents. Study and evaluation of the active agents of this invention continues.

Contrary to prior agents (such as Heparin and coumadin), the active agents of the present invention appear to affect both blood coagulation factors and platelet aggregation. Conveniently and preferably, measurements of blood factors are carried out periodically, such as every 3 to 7 days, on a patient under-going treatment under the practice of this invention.

An active agent can be given orally, in the form of a capsule or tablet, or in the form of a solution (e.g. aqueous). Also, an active agent can be injected in the form of an aqueous solution.

A particularly preferred antithrombotic field of use is in post-operative treatment, as when arteries or deep veins may be involved in, or threatened by, a thrombotic condition.

By way of explanation, as those familiar with mammalian anatomy appreciate, the venous system is the lower extremieites consists of superficial and deep veins. Because of the manner in which the deep

veins interconnect and supply blood to the heart and lungs, a thrombus occurring in the deep veins, but not in the superficial veins, can become the source of a blood clot which is moved through the veins and becomes lodged in the lungs, resulting in a pulmonary embolus, which can have obvious catastrophic effects (including causing death). Examples of deep veins include the iliac, the femoral, the popliteal and the calf veins. The prevention of pulmonary emboli following surgery affecting the deep veins in the lower extremities is a significant medical problem. One solution to this problem is to prevent thrombi from occurring and/or developing in deep veins. To achieve this, active agents of this invention appear to be well suited. Thus, in carrying out this invention, one may achieve a symptomatic and objective improvement of a deep vein thrombotic complication in a patient during post-operative care, inhibiting intravascular thrombus formation (including embolism).

In one preferred administration of compositions of this invention, an aqueous solution of 1 to 10 percent by weight of an active agent of this invention, preferably sodium bisulphite, is prepared. Then, this solution is orally consumed by a human or is injected at a total (or accumulated) dosage rate ranging from 1.0 to 50 mg per kg of body weight per day, more preferably in the form of at least two spaced doses per day, and still more preferably in the form of at least three spaced doses per day, each dose preferably being taken around a meal time. Instead, solid or encapsulated active agents may be orally consumed.

Because of a tendency for bisulphites to undergo oxidation when in aqueous solution, it is currently preferred to minimize contact of active agents with oxygen before use. It is preferred in practising this invention with inorganic alkali metal bisulphites to employ a solution which comprises, on a 100% by weight total solution basis:

(a) 1% to 10% of dissolved inorganic solids, and

(b) the balance up to 100% of any given solution being water. In such a solution, the dissolved inorganic solids can comprise on a calculated 100% by weight dry basis:

(a) at least 50% and preferably at least 90% of alkali metal bisulphite, and

(b) the balance up to 100% of inorganic compounds produced or producible by the oxidation of alkali metal bisulphite.

The water used in such a solution is preferably purified (e.g., filtered, deionized or distilled). After preparation, the solution is preferably stored in a closed container to reduce oxidation.

Such an aqueous solution can be directly used in carrying out this invention, in which case such a solution can be dispensed dropwise or it can be encapsulated, for instance, and used as measured dosage units, as desired. For example, an aqueous solution containing 5 weight percent of sodium bisulphite can be injected into a patient, or it can be directly consumed by a patient as drops (e.g. from 5 to 9 drops per meal for each of the two or three meals eaten by such patient per day, depending upon an individual patient's body weight).

Symptomatic improvement in varicose veins and in haemorrhoids may be observable when using an active agent of this invention.

Since aqueous solutions of sulphur dioxide display a capacity to lengthen PT and PTT values at least to the extent indicated above, aqueous solutions of sulphur dioxide can be used in combination with, e.g. in admixture with, aqueous solutions of compounds forming the active treating agents of this invention.

A preferred method for treating a thrombotic condition in a mammal (including man), with a composition of the present invention, is characterized by:

(A) determining from a sample of blood removed from the mammal

(1) one or both of the blood coagulation factors consisting of prothrombin time and partial thromboplastin time and

(2) the platelet aggregation factor,

(B) when such factors fall within normal ranges for such mammal, administering to such mammal a pharmaceutical composition of the Invention at the dosage rate and for a time sufficient to inhibit the blood coagulation and platelet aggregation factors to an extent which is at least sufficient to inhibit thrombus development in the mammal but insufficient to cause spontaneous internal bleeding,

(C) at intervals during administration, removing further samples of blood from the mammal and determining the lengthened blood coagulation factor(s) and the inhibited platelet aggregation factor,

(D) adjusting administration of the composition so as to achieve and maintain predetermined desired values for the lengthened blood coagulation factor(s) and the inhibited platelet aggregation factor in the mammal, and

(E) continuing steps (B), (C) and (D) until the mammal has recovered from the thrombotic condition.

The compositions of the present invention can also be used *in vivo* or *in vitro* for inhibiting blood coagulation factors and for inhibiting platelet aggregation involving adding to blood and/or plasma derived from such blood an active agent of this invention.

The present invention is further illustrated by the following Examples. Reference may also be made to the disclosures, including specific Examples, contained in the aforesaid European Application No. 81302950.1 (EP—A—43274) and also European Application No. 82200090.7 (EP—A—55996) relating to various organic compounds, the latter having been divided out of the former, like the present disclosure. It is considered that a fuller understanding of the respective inventions can be obtained from the various disclosures when taken in conjunction.

Example A

A solution of sodium bisulphite was prepared by dissolving pharmaceutical grade (U.S.P) powdered sodium metabisulphite in distilled water at room temperature to form a 1% by weight solution.

This solution was put into plastics squeeze bottles, each having a volume of 50—52 ml. Each bottle was provided with a cap permitting dropwise dispensing of the solution from the bottle at an estimated rate of 15 drops per ml.

Example B

Example A was repeated, except that a 2.5% by weight solution was formed.

Example C

Example A was repeated, except that a 5% by weight solution was formed.

Example D

Example A was repeated, except that a 7.5% by weight solution was formed.

Example E

Example A was repeated except that a 10% by weight solution of potassium metabisulphite was prepared.

Example F

Baker analytical grade sodium bisulphite was placed in gelatin capsules so that each capsule contained 75 mg of sodium bisulphite.

Example G

Baker analytical grade sodium bisulphite was placed in gelatin capsules so that each capsule contained 50 mg of sodium bisulphite.

Example H

A solution of sulphur dioxide in water was prepared by bubbling a purified grade of sulphur dioxide through distilled water at room temperature to form a 2% by weight aqueous solution. The product was then titrated with alkali using phenolphthalein to verify its concentration.

Example I

A 1% by weight aqueous solution of sodium metabisulphite was prepared as in Example A. Purified $SO_2$ gas was then bubbled through the solution to give a concentration of $SO_2$ in the solution of 1% by weight.

Example 1

That sodium bisulphite is a platelet antiagregatory agent is shown by using a platelet aggregometer based on the technique of Born (above cited). It is found that this material in a concentration of 1 millimole (mM) abolishes platelet aggregation in human platelet-rich plasma occluded by optimal concentration of ADP, collagen, epinephrine, and arachidonic acid.

A similar effect is observed in rabbit platelet-rich plasma, except that slightly higher concentrations of this material are required to achieve an equivalent effect.

This indicates that this material does have a significant inhibitory effect *in vitro* on platelet aggregation. Based on these results, it appears that this material is suitable for use as an antithrombotic agent.

Example 2

Sodium bisulphite was evaluated as an anti-thrombotic agent *in vivo* using the indium-111 platelet labelling technique in rabbits (see the Wu et al article above cited). The material was administered by intravenous injection (72.80 milligrams per kilogram of animal body weight).

The results indicate that this material significantly reduces platelet thrombus formation on damaged aortic wall (see Table I below).

TABLE I
Platelet accumulation

| Rabbit | I. at damaged vessel wall cpm/gm* | % ** | II. at normal vessel wall cpm/gm* | % ** |
|---|---|---|---|---|
| A | 133,986 | 0.11 | 98,611 | 0.08 |
| B | 146,630 | 0.15 | 83,066 | 0.08 |
| Control | 1,167,820± | 0.44± | 72,140± | 0.03± |
| (means ± S.D. of 10 animals) | 21,310 | 0.08 | 39,040 | 0.02 |

*Count per minute per gram dry weight of tissue.
**%=percentage of injected radioactivity per gram dry weight of tissue.

The findings indicate that the active agent reduces accumulation of platelet thrombus at the damaged vessel wall by 70%.

Example 3

Sodium, potassium and ammonium bisulphites are found to prolong PT and PTT in a dose-related fashion. When added to human, rabbit and rat plasma *in vitro*, all these agents significantly prolong PT and PTT at a concentration of 0.5 mg/ml and the effects are found to be dose-related. The results are shown in Table II below.

The evaluation procedure used is that described in "Human Blood Coagulation, Haemostasis and Thrombosis", edited by Rosemary Biggs, published by Blackwell Scientific Publications, Oxford, England (2nd Edition), pages 670—705, 1976.

Example 4

The effect of U.S.P. grade sodium bisulphite on blood coagulation factors was evaluated by standard procedures (Biggs) and the results are shown in Table III below.

The work was replicated using chemical grade sodium bisulphite and the results are shown in Table IV below.

These results indicate that sodium bisulphite at a concentration of 0.5 mg per ml has a significant inhibitory effect on Factors IX, X, XI, XII, and VII. Factor II and Factor VIII are mildly affected by this agent. The effect is directly proportional to dose. At 5 mg/ml, the coagulation of human plasma is completely disrupted.

When this procedure was repeated using rabbit and rat plasma, similar results were observed, except that blood coagulation factors in these species are not as sensitive in their response to this agent.

When this procedure was repeated using human plasma, but employing potassium bisulphite and ammonium bisulphite, similar results were obtained.

Example 5

The anti-venous thrombotic effect of sodium bisulphite was evaluated in rabbits by using the $I^{125}$ fibrinogen technique described above.

Each rabbit was continuously infused with aqueous sodium bisulphite solution at the rate of 460 mg/kg per hr. (2.97 grams) (animal body weight 2.3 kg).

After 5 hours, the animal was sacrificed and the radioactive fibrinogen accumulated in the damaged jugular vein was determined. The radioactivity in the contra-lateral (undamaged) jugular vein was also determined, to serve as a control. Concurrently, another rabbit was subjected to the same procedure, but was given only a standard sodium chloride solution, as a control. It was found to have 7 times as much radioactivity as the counterpart infused with sodium bisulphite. Thus, sodium bisulphite shows a definite positive effect in decreasing thrombus formation in the jugular vein.

TABLE II
Activities of various active agents

| Example designation | Active agent (0.5/ml) | PT (seconds) Exp. 1 C | PT (seconds) Exp. 1 A | PT (seconds) Exp.2 C | PT (seconds) Exp.2 A | PTT (seconds) Exp. 1 C | PTT (seconds) Exp. 1 A | PTT (seconds) Exp. 2 C | PTT (seconds) Exp. 2 A |
|---|---|---|---|---|---|---|---|---|---|
| | Test sequence A | | | | | | | | |
| 7 | Sodium metabisulphite | 13.4 | 18.5 | 14.1 | 21.9 | 42.1 | 76.9 | 50.5 | 80.3 |
| 7.2 | Sodium sulphite | 13.4 | 16.2 | 14.1 | 17.7 | 42.1 | 66.1 | 50.5 | 80.7 |
| 7.3 | Ammonium sulphite | 13.0 | 14.7 | 14.1 | 16.9 | 44.7 | 56.5 | 50.5 | 65.2 |
| 7.4 | Sodium bisulphite | 13.9 | 22.1 | 14.1 | 23.2 | 53.8 | 128.2 | 50.5 | 121.9 |
| 7.5 | Potassium metabisulphite | 13.9 | 18.3 | 14.1 | 22.0 | 53.8 | 77.6 | 50.5 | 111.3 |
| | Test sequence B | | | | | | | | |
| 7.6 | Sodium sulphite | 13.1 | 16.8 | 13.5 | 17.7 | 44.0 | 74.8 | 44.5 | 82.1 |
| 7.7 | Sodium bisulphite | 13.5 | 25.5 | — | — | 44.0 | 128.2 | 44.5 | 162.6 |
| 7.8 | Potassium metabisulphite | 13.5 | 20.4 | — | — | 44.0 | 91.6 | 44.5 | 105.2 |

C=Control
A=Active agent

TABLE III
Effects of solution D on coagulation in vitro

| Coagulation parameters* | Control | Sodium bisulphite concentration (mg/ml) 0.01 | 0.05 | 0.1 | 0.5 | 1.0 | 5.0 |
|---|---|---|---|---|---|---|---|
| PT (sec)$^S$ | 34.67 | 33.37 | 34.00 | 34.85 | 42.73 | 85.33 | >150 |
| PTT (sec)$^S$ | 12.20 | 12.33 | 12.43 | 12.85 | 15.43 | 22.63 | >150 |
| TT (sec)$^S$ | 12.00 | 12.07 | 12.10 | 12.00 | 13.97 | 16.08 | 34.3 |
| Fibrogen (mg%) | 218 | 222 | 228 | 217 | 190 | 185 | 130 |
| Prothrombin (%) | 87 | 79 | 80 | 72 | 40 | 26 | 5 |
| Factor V (%) | 75 | 74 | 72 | 80 | 70 | 60 | 54 |
| F VII (%) | 73 | 67 | 57 | 51 | 23 | 8 | 1 |
| F VIII (%) | 57 | 62 | 68 | 55 | 58 | 29 | 11 |
| V IX (%) | 66 | 60 | 52 | 56 | 4 | 3 | <1 |
| F X (%) | 78 | 73 | 68 | 66 | 31 | 11 | 3 |
| F XI (%) | 117 | 98 | 81 | 58 | 27 | 4 | <1 |
| F XII (%) | 86 | 94 | 87 | 79 | 17 | 3 | <1 |

*Values represent mean of 5 determinations in duplicate
Abbreviations: PT: Prothrombin Time; PTT: Partial Thromboplastin Time; TT: Thrombin Time
Methods for PT, PTT, TT and Factor assay are standard

A second rabbit, similarly evaluated along with a control rabbit, displayed similar results. Other rabbits, similarly evaluated, died before the experiment was completed, apparently because of overdosing with this active agent.

Example 6

A variety of agents were coded and evaluated under a code designation. Among these compounds, thiouracil, mercaptosuccinic acid, thiosemicarbazide, phenothiazine and sodium thiosulphate were included, so as to compare sodium bisulphite with certain compounds mentioned in USP 4,148,855.

After determination of PT and PTT values, the coded compounds were decoded. The bisulphites and sulphites showed an effect *in vitro* and only one other compound (sodium formaldehyde bisulphite) was effected in increasing PT and PTT values.

The results are shown in Table V, where mean values of 5 replications in each case are reported. The findings demonstrate the reliability of the test method. This procedure was repeated with some additional compounds on two different days using a different control on each day. The results are shown in Table VI below. These data indicate that the active moieties are probably the bisulphite and sulphite groups.

Example 7

Five rats, each in a separate cage, were given a bait containing 0.5 percent by weight of sodium bisulphite. The bait consisted of cracked grains (wheat, corn, millet and corn meal). The amounts of bait eaten were summarized for an eleven day period. The first day determination of PT showed that three of the rats had an increase of PT values with no apparent change in PTT (Table VII).

TABLE IV

Effects of chemical grade sodium bisulphite on blood coagulation in vitro

| Coagulation parameters* | Control | Sodium bisulphite concentration (mg/ml) 0.01 | 0.05 | 0.1 | 0.5 | 1.0 | 5.0 |
|---|---|---|---|---|---|---|---|
| PTT (sec) | 34.67 | 32.80 | 33.77 | 34.40 | 44.87 | 78.46 | >150 |
| PT (sec) | 12.20 | 12.30 | 12.47 | 12.90 | 16.10 | 22.38 | >150 |
| TT (sec) | 12.00 | 12.20 | 12.30 | 12.15 | 14.53 | 16.23 | 35.5 |
| Fibrinogen (mg%) | 218 | 228 | 245 | 218 | 182 | 184 | 119 |
| Prothrombin (%) | 87 | 88 | 82 | 75 | 34 | 25 | 8 |
| Factor V | 75 | 70 | 68 | 68 | 64 | 66 | 60 |
| F VII | 73 | 62 | 63 | 53 | 17 | 7 | 2 |
| F VIII | 57 | 58 | 55 | 56 | 55 | 31 | 13 |
| F IX | 66 | 49 | 44 | 40 | 5 | 1 | <1 |
| F X | 78 | 80 | 69 | 66 | 22 | 6 | 4 |
| F XI | 117 | 83 | 82 | 55 | 24 | 2 | <1 |
| F XII | 86 | 104 | 102 | 75 | 25 | 9 | <1 |

*Values represent mean of 5 determinations in duplicate
Abbreviations: PT: Prothrombin Time; PTT: Partial Thromboplastin Time; TT: Thrombin Time
Methods for PT, PTT, TT and Factor assay are standard

TABLE V
Effects of coded agents (0.5 mg/ml)

| L.C. lab | Unknown reagents (Code 1) | No | PT (sec) | No | PTT (sec) | |
|---|---|---|---|---|---|---|
| | Control | 5 | 12.92± | 5 | 43.38± | |
| | $A_2$ | 5 | 14.60± | 5 | 48.70± | Mercaptosuccinic acid |
| | $A_3$ | 4 | 14.95± | 3 | 53.00± | Dihydroxymalcic acid 2 $H_2O$ |
| | $SO_2$ | 5 | 14.94± | 5 | 49.22± | Sodium formaldehyde sulphoxalate |
| | $SO_3$ | 5 | 19.50± | 5 | 58.60± | Sodium formaldehyde bisulphite |
| | $SO_4$ | 5 | 14.02± | 5 | 49.60± | Sodium diethyl dithiocarbamate |
| | $SI_1$ | 5 | 19.34± | 5 | 83.84± | Potassium metabisulphite |
| | $SI_2$ | 5 | 16.14± | 5 | 64.62± | Sodium sulphite |
| | $SI_4$ | 5 | 23.06± | 5 | 114.80± | Sodium bisulphite |
| | Control $A_1$ | | 13.10 | | 44.7 | |
| | $A_4$ | | 12.70 | | 51.8 | L-Ascorbic acid |
| | $N_1$ | | 13.1 | | 47.5 | D-(−)-Ascorbic acid |
| | $N_2$ | | 12.8 | | 45.4 | Thiouracil |
| | $N_3$ | | 12.7 | | 43.9 | Carbohydrate |
| | $N_4$ | | 13.0 | | 47.6 | Thiosemicarbazide |
| | $SO_1$ | | 12.9 | | 45.4 | Sodium p-toluene sulphinate |
| | $SI_3$ | | 13.6 | | 43.0 | Sodium phosphate |
| | $SI_5$ | | 13.4 | | 44.2 | Sodium hypophosphate |
| | $SI_6$ | | 13.5 | | 42.4 | Sodium thiosulphate |

TABLE VI
Effects of various agents (0.5 mg/ml)

| Reagents | No exp | PT (sec) | No exp | PTT (sec) |
|---|---|---|---|---|
| Control | 5 | 13.40± | 4 | 42.6± |
| $Na_2S_2O_5$ | 5 | 19.56± | 4 | 77.75± |
| $NaHSO_3$ | 5 | 22.42± | 4 | 120.35± |
| Na Sulphide | 5 | 21.12± | 4 | 78.50± |
| $Na_2SO_3$ | 5 | 16.38± | 4 | 66.53± |
| $(NH_4)_2SO_3$ | 5 | 16.08± | 4 | 58.80± |
| Na-formaldehyde—$HSO_3$ | 5 | 19.68± | 3 | 56.30± |
| $K_2H_2SO_5$ | 5 | 18.76± | 4 | 80.60± |
| Control | — | 13.40 | — | 42.10 |
| Na-phosphite | 1 | 12.9 | — | — |
| Na-hydrosulphite | 2 | 14.4 | — | 45.2 (39.0) |
| mecaptosuccinic acid | 1 | 14.7 | 1 | 52.6 |
| Na-thiosulphate | 1 | 13.5 | 1 | 46.2 |
| $Na_2SO_4$ | 1 | 13.6 | 1 | 44.2 |
| Na-diethylthiocarbonate | 1 | 13.6 | 1 | 48.1 |
| Na-Hypophosphite | 1 | 13.9 | 1 | 47.19 (53.8) |
| Na-formaldehyde sulphoxalate | 1 | 13.8 | 1 | 45.1 (53.8) |

TABLE VII
Five 250g rats fed bait containing 0.5% sodium bisulphite

| Days | Rat 1 g bait eaten | Rat 2 g bait eaten | Rat 3 g bait eaten | Rat 4 g bait eaten | Rat 5 g bait eaten |
|---|---|---|---|---|---|
| 1 day | 27.8 | 25.9 | 26.7 | 28.0 | 27.0 |
| 2 days | Died | 27.9 | 17.3 | Died | 8.0 |
| 3 days | | 19.2 | 19.4 | | 11.4 |
| 4 days | | 18.9 | 10.1 | | 10.4 |
| 9 days | | 26.8 | 27.8 | | 25.7 |
| 10 days | | 22.7 | 26.2 | | 26.3 |
| 11 days | | 23.8 | 21.0 | | 22.8 |

| First day blood factor | Rat 1 PT | Rat 1 PTT | Rat 2 PT | Rat 2 PTT | Rat 3 PT | Rat 3 PTT | Rat 4 PT | Rat 4 PTT | Rat 5 PT | Rat 5 PTT |
|---|---|---|---|---|---|---|---|---|---|---|
| | 26% increase | 150 | 24% increase | 150 | 28% increase | 150 | — | — | 27 | 70 |

To evaluate the effect of sodium bisulphite in human use (this agent being known to be safe for human consumption), certain patients received this agent. The case history and observations are summarized individually below.

In each case, each patient was provided with a bottle of Solution D, unless otherwise noted, and was instructed to dose himself (or herself, as the case may be) from the bottle so provided at the rate of seven drops to be taken orally with each of his (her) three daily meals. When the contents of one such bottle were thus gradually consumed by an individual patient, another was provided.

Case I

A man now aged 33, height 1.78 m (5'10"), weight 88.8 kg, had a myocardial infarction at the age of 29. He had been treated with heparin as an anticoagulant and also with vasodilator agents. It was felt that he had coronary atherosclerosis and hypertension. He was also on a special low-salt, low-fat diet.

This man improved symptomatically in the patient's estimation after about 2 weeks of treatment with Solution D. A gradual lowering of his heparin dose was achieved and also of his vasodilator medicines. With continuance of the treatment, this man has been enjoying good health for about 3 years. It is reported that after an initial period of use of Solution D as described, this man's dosage rate was increased to 8 drops taken three times a day.

Case II

A women, now aged 64, height 1.65 m (5'5"), weight 86.3 kg, had a long history of severe varicose veins in her legs, and the right leg had become so swollen that she could scarcely walk.

After about 1 month of treatment with Solution D, the swollen leg returned to normal, and her varicose condition improved as demonstrated by reduced size of the varices. After about 4 years of continuous use of Solution D, no recurrence of her original condition has resulted.

Case III

A man, aged 72 at death, height 1.83 m (6'), weight 86.4 kg, suffered an injury to his leg complicated by an embolus, which apparently had its inception in the leg, moved to the brain, and paralyzed the right side of his body. Subsequently, he began to have thrombophlebitis in both his legs.

After use of Solution D was started, his phlebitis gradually disappeared. Beneficial results began to be observed in about two weeks. However, his hemiparesis did not regress; he continued to be bed-ridden. It is reported that after an initial period of use of Solution D as described, his dosage rate was increased to 9 drops taken three times a day. After almost three years of continuously using Solution D, he had not experienced any return of phlebitis to his legs. However, this man died from a kidney infection.

Case IV

A man, now aged 79, height 1.80 m (5'11"), weight 82 kg, had mild hypertension, mild arthritis, mild arrhythmia, haemorrhoids, dyspepsia, cyanotic fingers and toes, mild diabetes, general malaise and lassitude. After his gall bladder had been removed at age 66, his digestion deteriorated. His diabetes was acquired shortly before treatment with sodium bisulphite began and has been controlled continuously since with Diabinese (500 mg daily).

After oral ingestion of aqueous solutions of sodium bisulphite of various concentrates (Solutions A, B, C or D) at dose rates varying from to 20 drops per meal, all of the above-identified conditions improved (except for diabetes) within 2—3 months. Thereafter they gradually disappeared and never recurred. After about seven years of continuous experimental use, the man remains in good health, and is alert and vigorous with excellent colour.

After about 3—4 years of use, the man found that he had a prolonged blood clotting time whereupon he reduced his dosage rate somewhat to about 8 drops per meal per day of Solution D and his blood clotting time then normalized.

Samples of about 4 cc each of his blood were prepared. To each of these was added about 10 drops of a 10 weight percent solution of sodium bisulphite in distilled water. Each sample was then sealed into a glass vial. After about 2½ years of storage at ambient temperatures, these blood samples have not coagulated.

At the time when prolonged clotting time was observed, the man was orally taking about 7 to 10 drops per meal per day of Solution E and his clotting time was estimated to have increased from about 5—9 minutes to 10 to 20 minutes.

Before taking this medication, this same man also had a blood cholesterol value which oscillated between 200 and 220 mg %. After about 5 years of taking this medication as described above, this man was found to have a blood cholesterol value of about 144 mg %, which value this man continues to keep through continued treatment by sodium bisulphite solutions as described above.

Case V

A man reported to be of average weight, had been in an automobile accident about 20 years previously, as a result of which his pelvis was broken and his legs were broken in many places. Then and now, the man smokes cigarettes heavily. Over a year after the accident, when the man had slowly begun to regain use of his legs without crutches, clotting problems in his leg veins developed. The main vein in his

left leg was replaced with a plastic vein which his body rejected. A second plastic vein was implanted and later blood clotting rendered it useless. A third plastic vein was implanted which has been effective for many years down to the present time. His sciatic nerve was damaged during the third vein implant operation. However, his left leg gradually became difficult to use and displayed severe swelling and paresthesia. His right leg also had some swelling problems.

Seven years after this third plastic vein had been in place, use of Solution C was started at a rate of about 7 drops with each of three daily meals. Gradually, swelling was reduced to normal in both legs and the parasthesis disappeared.

After preliminary treatment with Solution C, Solution D was substituted for Solution C at about the dose rate (7 drops with each of three daily meals). Physiotherapy is being used. Use of Solution C has not improved or changed the damage to his sciatic nerve, but walking has become easier.

**Claims for the Contracting States: BE, CH, DE, FR, GB, IT, LI, LU, NL, SE**

1. A pharmaceutical composition for treating an actual incipient thrombotic condition in a mammal, characterized in that it contains at least one carrier substance and, as the only active treating agent, at least one inorganic compound selected from alkali metal, alkaline earth metal and ammonium sulphites and bisulphites and sulphurous acid and the composition is in the form of:—

(a) a gelatin capsule, or

(b) an aqueous solution containing 1 to 10% by weight of the active treating agent contained in a drop dispenser.

2. A liquid pharmaceutical composition for treating an actual or incipient thrombotic condition in a mammal, characterized in that it contains at least one carrier substance and, as the only active treating agent, at least one inorganic compound selected from alkali metal, alkaline earth metal and ammonium sulphites and bisulphites and sulphurous acid and said composition is contained within a drop dispenser bearing instructions to administer the composition in such manner as to provide a dosage rate of 1 to 100 milligrams per kilogram body weight per day for the purpose of treating an actual or incipient thrombotic condition in a mammal.

3. A pharmaceutical composition as claimed in Claim 2, wherein said dosage rate is 1 to 50 mg/kg.

4. A pharmaceutical composition as claimed in Claim 3, wherein said dosage rate is 20 to 50 mg/kg.

5. A pharmaceutical composition as claimed in any one of Claims 1 to 4, wherein the active treating agent is an alkali metal or ammonium sulphite or bisulphite.

6. A pharmaceutical composition as claimed in any one of the preceding claims, wherein the active agent is a bisulphite.

7. A pharmaceutical composition as claimed in Claim 5, wherein the active treating agent is sodium bisulphite.

8. A pharmaceutical composition as claimed in any one of Claims 1, 5, 6 and 7 wherein the composition is in the form of a gelatin capsule.

9. A pharmaceutical composition as claimed in any one of Claims 1 to 7, wherein the composition is in the form of an aqueous solution containing 1 to 10% by weight of the active treating agent contained in a drop dispenser.

10. A pharmaceutical composition as claimed in Claim 5, wherein the active treating agent is an alkali metal bisulphite and the composition is in the form of an aqueous solution containing 1 to 10% dissolved inorganic solids and the balance up to 100% water, at least 50% of the dissolved solids being alkali metal bisulphite and the balance of the dissolved solids being compounds produced or producible by oxidation of the alkali metal bisulphite.

11. A pharmaceutical composition as claimed in Claim 10, wherein the alkali metal bisulphite constitutes at least 90% of the dissolved inorganic solids.

12. A pharmaceutical composition as claimed in any one of the preceding claims, wherein the composition is in the form of an aqueous solution containing dissolved sulphur dioxide (i.e. sulphurous acid) and at least one other of the active treating agents.

13. The use of alkali metal, alkaline earth metal or ammonium sulphite or bisulphite in the manufacture of a medicament for the treatment of an actual or incipient thrombotic condition in a human patient.

**Claims for Contracting State: AT**

1. A method of preparing pharmaceutical product for treating an actual or incipient thrombotic condition in a mammal, characterized in that it comprises mixing at least one carrier substance and, as the only active treating agent, at least one inorganic compound selected from alkali metal, alkaline earth metal and ammonium sulphites and bisulphites and sulphurous acid to form a pharmaceutical composition and either

(a) filling a gelatin capsule with the composition, or

(b) when the composition is an aqueous solution containing 1 to 10% by weight of the active treating agent, filling a drop dispenser with the solution.

2. A method of preparing a pharmaceutical product for treating an actual or incipient thrombotic

condition in a mammal, characterized in that it comprises mixing at least one liquid carrier substance and, as the only active treating agent, at least one inorganic compound selected from alkali metal, alkaline earth metal and ammonium sulphites and bisulphites and sulphurous acid to form a liquid pharmaceutical composition and filling with said composition a drop dispenser bearing instructions to administer the composition in such a manner as to provide a dosage rate of 1 to 100 milligrams per kilogram body weight per day for the purpose of treating an actual or incipient thrombotic condition in a mammal.

3. A method as claimed in Claim 2, wherein said dosage rate is 1 to 50 mg/kg.

4. A method as claimed in Claim 3, wherein said dosage rate is 20 to 50 mg/kg.

5. A method as claimed in any one of Claims 1 to 4, wherein the active treating agent is an alkali metal or ammonium sulphite or bisulphite.

6. A method as claimed in any one of the preceding claims, wherein the active agent is a bisulphite.

7. A method as claimed in Claim 5, wherein the active treating agent is sodium bisulphite.

8. A method as claimed in any one of Claims 1, 5, 6 and 7, wherein a gelatin capsule is filled with the composition.

9. A method as claimed in any one of Claims 1 to 7, wherein a drop dispenser is filled with the composition is in the form of an aqueous solution containing 1 to 10% by weight of the active treating agent.

10. A method as claimed in Claim 5, wherein the active treating agent is an alkali metal bisulphite and the composition is in the form of an aqueous solution containing 1 to 10% dissolved inorganic solids and the balance up to 100% water, at least 50% of the dissolved solids being alkali metal bisulphite and the balance of the dissolved solids being compounds produced or producible by oxidation of the alkali metal bisulphite.

11. A method as claimed in Claim 10, wherein the alkali metal bisulphite constitutes at least 90% of the dissolved inorganic solids.

12. A method as claimed in any one of the preceding claims, wherein the composition is in the form of an aqueous solution containing dissolved sulphur dioxide (i.e. sulphurous acid) and at least one other of the active treating agents.

**Patentansprüche für die Vertragsstaaten: BE, CH, DE, FR, GB, IT, LI, LU, NL, SE**

1. Pharmazeutische Zusammensetzung zur Behandlung von akuter oder inzipienter Thrombose bei einem Säuger, dadurch gekennzeichnet, daß sie mindestens eine Trägersubstanz und als als einziges aktives Behandlungsmittel mindestens eine anorganische Verbindung ausgewählt aus Alkalimetall-, Erdalkalimetall-und Ammoniumsulfiten und -bisulfiten und schwefliger Säure enthält und die Zusammensetzung als

(a) Gelatinekapsel oder

(b) wässrige Lösung mit einem Gehalt von 1 bis 10 Gew.% des aktiven Behandlungsmittels in einem Tropfenspender vorliegt.

2. Flüssige pharmazeutische Zusammensetzung zur Behandlung von akuter oder inzipienter Thrombose bei einem Säuger, dadurch gekennzeichnet, daß sie mindestens eine Trägersubstanz und als einziges aktives Behandlungsmittel mindestens eine anorganische Verbindung ausgewählt aus Alkalimetall-, Erdalkalimetall- und Ammoniumsulfiten und -bisulfiten und schwefliger Säure enthält und die Zusammensetzung in einem Tropfenspender enthalten ist, der eine Gebrauchsanweisung trägt, die angibt, daß die Zusammensetzung in der Weise zu verabreichen ist, daß eine Dosierungsmenge von 1 bis 100 mg/kg Körpergewicht pro Tag zur Behandlung von akuter oder inzipienter Thrombose erreicht wird.

3. Pharmazeutische Zusammensetzung nach Anspruch 2, dadurch gekennzeichnet, daß die Dosierungsmenge 1 bis 50 mg/kg beträgt.

4. Pharmazeutische Zusammensetzung nach Anspruch 3, dadurch gekennzeichnet, daß die Dosierungsmenge 20 bis 50 mg/kg beträgt.

5. Pharmazeutische Zusammensetzung nach einem der vorangehenden Ansprüche 1 bis 4, dadurch gekennzeichnet, daß das aktive Behandlungsmittel ein Alkalimetall- oder Ammoniumsulfit oder -bisulfit ist.

6. Pharmazeutische Zusammensetzung nach jeglichem der vorangehenden Ansprüche, dadurch gekennzeichnet, daß das aktive Behandlungsmittel ein Bisulfit ist.

7. Pharmazeutische Zusammensetzung nach Anspruch 5, dadurch gekennzeichnet, daß das aktive Behandlungsmittel Natriumbisulfit ist.

8. Pharmazeutische Zusammensetzung nach einem der vorangehenden Ansprüche 1, 5, 6 und 7, dadurch gekennzeichnet, daß die Zusammensetzung als Gelatinekapsel vorliegt.

9. Pharmazeutische Zusammensetzung nach den Ansprüchen 1 bis 7, dadurch gekennzeichnet, daß die Zusammensetzung als wässrige Lösung, die 1 bis 10 Gew.% des aktiven Behandlungsmittels enthält, in einem Tropfenspender vorliegt.

10. Pharmazeutische Zusammensetzung nach Anspruch 5, dadurch gekennzeichnet, daß das aktive Behandlungsmittel ein Alkalimetallbisulfit ist und die Zusammensetzung als wässrige Lösung vorliegt, die 1 bis 10 % gelöste anorganische Feststoffe enthält und bei der die Differenz bis zu 100 % Wasser ist, wobei mindestens 50% der gelösten Feststoffe Alkalimetallbisulfit und die Differenz der gelösten Feststoffe Verbindungen sind, die durch Oxydation des Alkalimetallbisulfits gebildet oder bildbar sind.

11. Pharmazeutische Zusammensetzung nach Anspruch 10, dadurch gekennzeichnet, daß das Alkalimetallbisulfit mindestens 90 % der gelösten anorganischen Feststoffe bildet.

12. Pharmazeutische Zusammensetzung nach jeglichem der vorangehenden Ansprüche, dadurch gekennzeichnet, daß die Zusammensetzung als wässrige Lösunge vorliegt, die gelöstes Schwefeldioxid (i.e. schweflige Säure) und mindestens ein weiteres der aktiven Behandlungsmittel enthält.

13. Verwendung von Alkalimetall-, Erdalkalimetall- oder Ammoniumsulfiten oder -bisulfiten zur Herstellung eines Arzneimittels zur Behandlung von akuter oder inzipienter Thrombose beim Menschen.

**Patentansprüche für den Vertragsstaat: AT**

1. Verfahren zur Herstellung eines pharmazeutischen Produktes zur Behandlung akuter oder inzipienter Thrombose bei einem Säuger, dadurch gekennzeichnet, daß man mindestens eine Trägersubstanz und als einziges aktives Behandlungsmittel mindestens eine anorganische Verbindung ausgewählt aus Alkalimetall-, Erdalkalimetall- und Ammoniumsulfiten und -bisulfiten und schwefliger Säure miteinander vermischt, um eine pharmazeutische Zusammensetzung herzustellen, und entweder

(a) Gelatinekapseln mit der Zusammensetzung füllt, oder

(b) wenn die Zusammensetzung als wässrige Lösung vorliegt, die 1 bis 10 Gew.% des aktiven Behandlungsmittels enthält, einen Tropfenspender mit der Lösung füllt.

2. Verfahren zur Herstellung eines pharmazeutischen Produktes zur Behandlung von akuter oder inzipienter Thrombose beim Säuger, dadurch gekennzeichnet, daß man mindestens eine flüssige Trägersubstanz und als einziges aktives Behandlungsmittel mindestens eine anorganische Verbindung ausgewählt aus Alkalimetall-, Erdalkalimetall- oder Ammonium-sulfiten und -bisulfiten und schwefliger Säure miteinander vermischt, um eine flüssige pharmazeutische Zusammensetzung herzustellen, und einen Tropfenspender mit der Zusammensetzung füllt, der eine Gebrauchsanweisung trägt, die angibt, daß die Zusammensetzung in der Weise zu verabreichen ist, daß eine Dosierungsmenge von 1 bis 100 mg/kg Körpergewicht pro Tag zur Behandlung von akuter oder inzipienter Thrombose bei einem Säuger erreicht wird.

3. Verfahren nach Anspruch 2, dadurch gekennzeichnet, daß die Dosierungsmenge 1 bis 50 mg/kg beträgt.

4. Verfahren nach Anspruch 3, dadurch gekennzeichnet, daß die Dosierungsmenge 20 bis 50 mg/kg beträgt.

5. Verfahren nach einem der Ansprüche 1 bis 4, dadurch gekennzeichnet, daß das aktive Behandlungsmittel ein Alkalimetall- oder Ammoniumsulfit oder -bisulfit ist.

6. Verfahren nach jeglichem der vorangehenden Ansprüche, dadurch gekennzeichnet, daß das aktive Mittel ein Bisulfit ist.

7. Verfahren nach Anspruch 5, dadurch gekennzeichnet, daß das aktive Mittel Natriumbisulfit ist.

8. Verfharen nach jeglichem der Ansprüche 1, 5, 6 und 7, dadurch gekennzeichnet, daß man eine Gelatinekapsel mit der Zusammensetzung füllt.

9. Verfharen nach jeglichem der Ansprüche 1 bis 7, dadurch gekennzeichnet, daß man einen Tropfenspender mit der Zusammensetzung als einer wässrigenn Lösung füllt, die 1 bis 10 Gew.% des aktiven Behandlungsmittels enthält.

10. Verfahren nach Anspruch 5, dadurch gekennzeichnet, daß das aktive Behandlungsmittel ein Alkalimetallbisulfit ist und die Zusammensetzung als wässrige Lösung vorliegt, die 1 bis 10 % gelöste Feststoffe enthält und der die Differenz bis zu 100 % Wasser ist, wobei mindestens 50 % der gelösten Feststoffe Alkalimetallbisulfite sind und die Differenz der gelösten Feststoffe Verbindungen sind, die durch Oxydation des Alkalimetallbisulfits gebildet werden oder bildbar sind.

11. Verfahren nach Anspruch 10, dadurch gekennzeichnet, daß das Alkalimetallbisfulfit mindestens 90 % der gelösten anorganischen Feststoffe bildet.

12. Verfahren nach jeglichen der vorangehenden Ansprüche, dadurch gekennzeichnet, daß die Zusammensetzung als wässrige Lösung vorliegt, die gelöstes Schwefeldioxid (i.e. schweflige Säure) und mindestens ein weiteres der aktiven Behandlungsmittel enthält.

**Revendications Pour les Etats Contractants; BE, CH, DE, FR, GB, IT, LI, LU, NL, SE**

1. Composition pharmaceutique pour le traitement d'un état thrombotique établi ou débutant chez un mammifère, caractérisée en ce qu'elle contient au moins une substance véhicule et, en tant que seul agent actif de traitement, au moins un composé inorganique choisi parmi des sulfites et bisulfites de métaux alcalins, de métaux alcalinoterreux et d'ammonium et l'acide sulfureux, et en ce que la composition est sous la forme

(a) d'une capsule de gélatine ou

(b) d'une solution aqueuse contenant 1 à 10 % en poids de l'agent actif de traitement, contenue dans un distributeur goutte à goutte.

2. Composition pharmaceutique liquide pour le traitement d'un état thrombotique établi ou débutant chez un mammifère, caractérisée en ce qu'elle contient au moins une substance véhicule et, en tant que seul agent actif de traitement, au moins un composé inorganique choisi parm des sulfites et bisulfites de

métaux alcalins, de métaux alcalinoterreux et d'ammonium et l'acide sulfureux, et en ce que cette composition est contenue dans un distributeur goutte à goutte portant des instructions pour que la composition soit adminstrée avec une posologie de 1 à 100 mg/kg de poids corporel par jour en vue du traitement d'un état thrombotique établi ou débutant chez un mammifère.

3. Composition pharmaceutique selon la revendication 2, caractérisée en ce que la posologie est de 1 à 50 mg/kg.

4. Composition pharmaceutique selon la revendication 3, caractérisée en ce que la posologie est de 20 à 50 mg/kg.

5. Composition pharmaceutique selon l'une quelconque des revendications 1 à 4, caractérisée en ce que l'agent actif de traitement est un sulfite ou bisulfite de métal alcalin ou d'ammonium.

6. Composition pharmaceutique selon l'une quelconque des revendications 1 à 5, caractérisée en ce que l'agent actif est un bisulfite.

7. Composition pharmaceutique selon la revendication 5, caractérisée en ce que l'agent actif de traitement est le bisulfite de sodium.

8. Composition pharmaceutique selon l'une quelconque des revendications 1, 5, 6 ou 7, caractérisée en ce que la composition est sous la forme d'une capsule de gélatine.

9. Composition pharmaceutique selon l'une quelconque des revendications 1 à 7, caractérisé en ce que la composition est sous la forme d'une solution aqueuse contenant 1 à 10 % en poids de l'agent actif de traitement, contenue dans un distributeur goutte à goutte.

10. Composition pharmaceutique selon la revendication 5, caractérisé en ce que l'agent actif de traitement est un bisulfite de métal alcalin et en ce que la composition est sous la forme d'une solution aqueuse contenant 1 à 10 % de matières solides inorganiques dissoutes, le reste à 100% étant formé d'eau, au moins 50% des matières solides dissoutes étant constituées par un bisulfite de métal alcalin et le reste des matières solides dissoutes étant constitué par des composés formés ou formables par l'oxydation du bisulfite de métal alcalin.

11. Composition pharmaceutique selon la revendication 10, caractérisée en ce que le bisulfite de métal alcalin constitue au moins 90% des matières solides inorganiques dissoutes.

12. Composition pharmaceutique selon l'une quelconque des revendications 1 à 11, caractérisée en ce que la composition est sous la forme d'une solution aqueuse contenant du bioxyde de soufre (c'est-à-dire de l'acide sulfureux) dissous et au moins un autre des agents actifs de traitement.

13. Utilisation de sulfite ou bisulfite de métal alcalin, de métal alcalinoterreux ou d'ammonium dans la préparation d'un médicament pour le traitement d'un état thrombotique établi ou débutant chez un patient humain.

**Revendications Pour l'Etat Contractant: AT**

1. Procédé de préparation d'un produit pharmaceutique pour le traitement d'un état thrombotique établi ou débutant chez un mammifère, caractérisé en ce qu'il comprend les opérations consistant à mélanger au moins une substance véhicule et, en tant que seul agent actif de traitement, au moins un composé inorganique choisi parmi des sulfites et bisulfites de métaux alcalins, de métaux alcalinoterreux et d'ammonium et l'acide sulfureux, pour former une composition pharmaceutique et

(a) soit à remplir une capsule de gélatine avec la composition,

(b) soit, lorsque la composition est une solution aqueuse contenant 1 à 10 % en poids de l'agent actif de traitement, à remplir un distributeur goutte à goutte avec la solution.

2. Procédé de préparation d'un produit pharmaceutique pour le traitement d'un état thrombotique établi ou débutant chez un mammifère, caractérisé en ce qu'il comprend les opérations consistant à mélanger au moins une substance véhicule liquide et, en tant que seul agent actif de traitement, au moins un composé inorganique choisi parmi des sulfites et bisulfites de métaux alcalins, de métaux alcalinoterreux et d'ammonium et l'acide sulfureux, pour former une composition pharmaceutique liquide, et à remplir avec cette composition un distributeur goutte à goutte portant des instructions pour que la composition soit administrée avec une posologie de 1 à 100 mg/kg de poids corporel par jour en vue du traitement d'un état thrombotique établi ou débutant chez un mammifère.

3. Procédé selon la revendication 2, caractérisé en ce que la posologie est de 1 à 50 mg/kg.

4. Procédé selon la revendication 3, caractérisé en ce que la posologie est de 20 à 50 mg/kg.

5. Procédé selon l'une quelconque des revendications 1 à 4, caractérisé en ce que l'agent actif de traitement est un sulfite ou bisulfite de métal alcalin ou d'ammonium.

6. Procédé selon l'une quelconque des revendications 1 à 5, caractérisé en ce que l'agent actif est un bisulfite.

7. Procédé selon la revendication 5, caractérisé en ce que l'agent actif de traitement est le bisulfite de sodium.

8. Procédé selon l'une quelconque des revendications 1, 5, 6 ou 7, caractérisé en ce qu'une capsule de gélatine est remplié avec la composition.

9. Procédé selon l'une quelconque des revendications 1 à 7, caractérisé en ce qu'un distributeur goutte à goutte est rempli avec la composition qui est sous la forme d'une solution aqueuse contenant 1 à 10 % en poids de l'agent actif de traitement.

10. Procédé selon la revendication 5, caractérisé en ce que l'agent actif de traitement est un bisulfite de métal alcalin et en ce que la composition est sous la forme d'une solution aqueuse contenant 1 à 10 % de matières solides inorganiques dissoutes, le reste à 100% étant formé d'eau, au moins 50% des matières solides dissoutes étant constituées par un bisulfite de métal alcalin et le reste des matières solides dissoutes étant constitué par des composés formés ou formables par l'oxydation du bisulfite de métal alcalin.

11. Procédé selon la revendication 10, caractérisé en ce que le bisulfite de métal alcalin constitue au moins 90 % des matiéres solides inorganiques dissoutes.

12. Procédé selon l'une quelconque des revendications 1 à 11, caractérisé en ce que la composition est sous la forme d'une solution aqueuse contenant du bioxyde de soufre (c'est-a-dire de l'acide sulfureux) dissou et au moins un autre des agents actifs de traitement.